# EUROPÄISCHE PATENTSCHRIFT

(11) **EP 1 469 835 B1**
(45) Veröffentlichungstag und Bekanntmachung des Hinweises auf die Patenterteilung: **14.11.2007**
(21) Anmeldenummer: 03729426.1
(22) Anmeldetag: 07.01.2003
(51) Int. Cl.: A61K 9/16, A61K 9/00, A61K 9/20, C07C 229/26

(54) **STABILE SALZE VON O-ACETYLSALICYLSÄURE MIT BASISCHEN AMINOSÄUREN II**
STABLE SALTS OF O-ACETYLSALICYLIC ACID CONTAINING BASIC AMINO ACIDS II
SELS STABLES D'ACIDE O-ACETYLSALICYLIQUE CONTENANT DES ACIDES AMINES II BASIQUES

(30) Priorität: 18.01.2002 DE 10202019
(43) Veröffentlichungstag der Anmeldung: 27.10.2004
(73) Patentinhaber: Bayer HealthCare AG, 51368 Leverkusen (DE)
(72) Erfinder: ECKER, Felix, Morris Plains, NJ 07950 (US); BÜLLESBACH, Ralf, Hackettstown, NJ 07840 (US); LEDWOCH, Wolfram, 40764 Langenfeld (DE); FRANCKOWIAK, Gerhard, 42115 Wuppertal (DE); GESSNER, Uwe, 53819 Neunkirchen-Seelscheid (DE); PETERSEN-BRAUN, Marianne, 53639 Königswinter (DE)
(86) Internationale Anmeldenummer: PCT/EP2003/000059
(87) Internationale Veröffentlichungsnummer: WO 2003/059323

(56) Entgegenhaltungen:
- WO-A-02/05782
- US-A- 4 265 888
- US-A- 4 988 683

## Beschreibung

Die vorliegende Erfindung betrifft verbesserte Zusammensetzungen mit stabilen Salzen von o-Acetylsalicyclsäure mit basischen Aminosäuren, diese enthaltende Arzneimittel sowie deren Verwendung zur Herstellung von Arzneimitteln.

Die analgetische, antipyretische und antiinflammatorische Wirkung von Acetylsalicylsäure ist seit langem bekannt. So wird o-Acetylsalicylsäure als Analgetikum, Antipyretikum, Antirheumatikum, sowie als nichtsteroidaler entzündungshemmender Wirkstoff beispielsweise zur Behandlung von Arthritiden, Neuralgien und Myalgien eingesetzt.

Acetylsalicylsäure ist auch nach über 100 Jahren das Standardtherapeutikum in der Selbstmedikation zur Therapie von Schmerz und Fieber, insbesondere Kopfschmerzen, Migräne und erkältungsbedingten Begleitsymptomen wie Kopf-, Hals- und Gliederschmerzen.

Allerdings ist o-Acetylsalicylsäure nur begrenzt löslich und somit die Resorptionsgeschwindigkeit limitiert. Gerade bei Schmerzen, vor allem bei Migräne, ist für den therapeutischen Effekt ein rasches Anfluten des Wirkstoffs im Körper erwünscht und erforderlich. Bisher konnte dies nur durch geeignete Darreichungsformen, wie z.B. gepufferten Brausetabletten oder Kautabletten erreicht werden.

Eine Möglichkeit, schnell hohe Blutspiegel des Wirkstoffs zu erreichen, ist die Vergrößerung der Lösegeschwindigkeit des Wirkstoff selbst. Dies ist mit Salzen der Acetylsalicylsäure zu erreichen. Zudem ist bei längerer oraler Verabreichung eine gute Verträglichkeit der o-Acetylsalicylate hervorzuheben.

Bekannte Salze der Acetylsalicylsäure sind unter anderem Salze der Acetylsalicylsäure mit basischen Aminosäuren. Therapeutisch wird das Salz der Acetylsalicylsäure mit der Aminosäure Lysin eingesetzt. Bei dem gängigsten Medikament mit ASS-Lysinat handelt es sich um eine Darreichungsform zur parenteralen Applikation. Es ist unter dem Namen Aspisol® im Handel.

Durch die orale Gabe von ASS-Lysinat kann eine Anflutung des Wirkstoffs erreicht werden, die beinahe der Blutspiegelkurve einer Bolusinjektion entspricht. Dies ist in der Literatur beschrieben [Ch. Raschka, H. J. Koch, Perfusion 6 (2000), 13. Jahrgang, Verlag PERFUSION, Nürnberg]. Der Wirkstoff löst sich außergewöhnlich schnell auf und wird sofort vom Körper resorbiert.

Auf dem Markt ist ASS-Lysinat als orale Darreichungsform nur als Pulver/Granulat erhältlich (z.B. Delgesic®, Aspegic®). Bisher konnten feste orale Darreichungsformen nicht hergestellt werden. Die Stabilität dieser Formulierungen war auch zu gering, so dass die Haltbarkeit dieser Formulierungen zur Vermarktung nicht ausreichte.

Die geringe Stabilität der o-Acetylsalicylate ist auf eine dem Fachmann bekannte Rückreaktion des Produkts zu o-Acetylsalicylsäure und der entsprechenden Aminosäure zurückzuführen. Die Aminosäure reagiert dann mit der o-Acetylsalicylsäure unter Abspaltung der Acetylgruppe (Amidolyse) und Freisetzung von Salicylsäure. Die Anwesenheit von Salicylsäure in pharmazeutischen Präparaten ist jedoch unerwünscht und deshalb auf einen geringen, akzeptablen Wert zu begrenzen. Es ist bekannt, dass diese Abbaureaktion pH-abhängig ist [F. Moll, Arch. Pharm. 318 (1985), 120 - 127]. Eine Erniedrigung des pH-Werts führt zu einer verstärkten Protonierung der freigesetzten Aminosäure, so dass diese für die Folgereaktion mit der o-Acetylsalicylsäure nicht oder nur noch sehr eingeschränkt zur Verfügung steht. Dadurch wird die Amidolyse und somit die Freisetzung von Salicylsäure zurückgedrängt.

Außerdem sind auch formulierungstechnische Probleme zur berücksichtigen. Zwar lassen sich Acetylsalicylate gut kompaktieren, sie zeigen aber nur ein unzureichendes Fließverhalten und neigen stark zum Ansetzen an den Presswerkzeugen von Tablettenpressen. Diese Probleme werden durch Zugabe von FST (Fließ-, Schmier- und Trenn)-Mitteln, üblicherweise von Magnesiumstearat, gelöst. Aus der Literatur ist dem Fachmann jedoch bekannt, dass Acetylsalicylsäure und Magnesiumstearat inkompatibel sind (P. C. Schmidt, Wirk- und Hilfsstoffe für Rezeptur, Defektur und Großherstellung, Wiss. Verl.-Ges., Stuttgart 1999). Teilweise können die Probleme umgangen werden, wenn der Wirkstoff in Hartgelatinekapseln abgefüllt wird. Dies ist in diesem Fall ebenfalls problematisch, da Acetylsalicylsäure und Gelatine bekanntermaßen unverträglich sind.

US 4,988,683 beschreibt Tabletten enthaltend DL-Lysinacetylsalicylat.

In der internationalen Patentanmeldung mit dem Aktenzeichen PCT/EP01/07669 (WO 02/05782 A) werden Salze von o-Acetylsalicylsäure mit basischen Aminosäuren beschrieben, die eine erhöhte Stabilität aufweisen und deshalb die Nachteile der bisher bekannten o-Acetylsalicylate hinsichtlich Lagerung und/oder Sterilisierbarkeit nicht aufweisen. Die Salze werden nach einem besonderen Verfahren hergestellt und zeichnen sich bei einer mit einem Malvern 2600D-Gerät unter Standardbedingungen gemessenen Korngrößenverteilung durch eine mittlere Korngröße oberhalb einer Korngröße von 160 µm und einen Anteil von mehr als 60 % der Teilchen mit einer Korngröße in einem Bereich von 100 bis 200 µm aus. Sie können einen gewissen Gehalt an zugegebenem Glycin aufweisen und eignen sich als Arzneimittel sowie zu deren Herstellung, beispielsweise in Form von Tabletten, Kautabletten oder Kapseln zur oralen Applikation.

Die in der internationalen Patentanmeldung PCT/EP01/07669 (WO 02/05782 A) beschriebenen o-Acetylsalicylate unterscheiden sich in ihrer Komgrößenanalyse deutlich und vorteilhaft von den bisher bekannten o-Acetylsalicylaten. Die Verteilung der Korngrößen ist bei diesen o-Acetylsalicylaten enger und die mittlere Korngröße zu höheren Partikelabmessungen verschoben. Dies bedeutet, dass diese o-Acetylsalicylate aus größeren und einheitlicher geformten (gewachsenen) Kristallen bestehen. Neben einer engeren Korngrößenverteilung und höheren mittleren Korngröße zeigen diese o-Acetylsalicylate zusätzlich eine gut ausgeprägte Kristallstruktur. Im Vergleich hierzu besitzt das gewerblich erhältliche o-Acetylsalicylat Aspisol® eine deutlich schlechter ausgeprägte Kristallstruktur. Diese vorteilhaften Eigenschaften dieser o-Acetylsalicylate führen dazu, dass der Restfeuchtegehalt dieser o-Acetylsalicylate außerordentlich niedrig gehalten und somit die vorstehend beschriebene Rückreaktion der o-Acetylsalicylate zu o-Acetylsalicylsäure und der entsprechenden Aminosäure unterdrückt werden kann. Dies ist um so erstaunlicher, da o-Acetylsalicylate an sich als hygroskopisch beschrieben sind. Diese o-Acetylsalicylate weisen aber eine verminderte Hygroskopie auf und sind bei gleicher Temperatur deutlich stabiler als herkömmliche Acetylsaliclyate mit höherem Restfeuchtegehalt.

Es wurde nun gefunden, dass sich die in der internationalen Patentanmeldung PCT/EP01/07669 (WO 02/05782 A) beschriebenen o-Acetylsalicylate unter Erhalt ihrer Stabilitätsvorteile wesentlich besser zu einzeln dosierten festen oralen Darreichungsformen mit ausreichender Stabilität verarbeiten lassen, wenn ihnen zur Verbesserung der Fließeigenschaften ein Fließmittel zugesetzt wird und/oder sie einem Granulationsverfahren unterzogen werden.

Gegenstand der Erfindung ist daher eine Zusammensetzung, umfassend ein Salz von o-Acetylsalicylsäure mit einer basischen Aminosäure, das bei einer mit einem Malvern 2600D-Gerät unter Standardbedingungen gemessenen Korngrößenverteilung eine mittlere Korngröße oberhalb einer Korngröße von 160 µm und einen Anteil von mehr als 60 % der Teilchen mit einer Korngröße in einem Bereich von 100 bis 200 µm aufweist, dadurch gekennzeichnet, dass die Zusammensetzung zusätzlich ein Fließmittel umfasst und/oder mittels Trockengranulation granuliert ist, wobei mikrokristalline Cellulose als Fließmittel ausgenommen ist.

Gegenstand der Erfindung ist weiterhin ein Arzneimittel, enthaltend mindestens eine erfindungsgemäße Zusammensetzung.

Gegenstand der Erfindung ist schließlich auch die Verwendung von erfindungsgemäßen Zusammensetzung zur Herstellung von Arzneimitteln für bestimmte Anwendungen.

Die vorliegende Erfindung wird durch die beiliegenden Zeichnungen näher erläutert.

Es zeigen:
- Fig. 1: Eine graphische Darstellung der Korngrößenverteilung des nach Beispiel 1 hergestellten o-Acetylsalicylats im Vergleich zur Korngrößenverteilung eines handelsüblichen o-Acetylsalicylats (Aspisol®).
- Fig. 2: Die Integrale der in Fig. 1 dargestellten Kurven der Korngrößenverteilungen für das erfindungsgemäße Beispiel 1 und Aspisol®.
- Fig. 3: Eine graphische Darstellung der Stabilität von erfindungsgemäßen Kautabletten gemäß Beispiel 4. Es ist die Veränderung des Gehalts an freier Salicylsäure (in %) über eine Lagerdauer von 12 Wochen bei einer Temperatur von 25°C und einer relativen Luftfeuchte von 60 % angegeben.
- Fig. 4: Eine graphische Darstellung der Stabilität von erfindungsgemäßen Tabletten gemäß Beispiel 5. Es ist die Veränderung des Gehalts an freier Salicylsäure (in %) über eine Lagerdauer von 12 Wochen bei einer Temperatur von 25°C und einer relativen Luftfeuchte von 60 % angegeben.
- Fig. 5: Eine graphische Darstellung der Stabilität von erfindungsgemäßen Kapseln gemäß Beispiel 6. Es ist die Veränderung des Gehalts an freier Salicylsäure (in %) über eine Lagerdauer von 12 Wochen bei einer Temperatur von 25°C und einer relativen Luftfeuchte von 60 % angegeben.

Erfindungsgemäß bevorzugt sind Zusammensetzungen, in denen das darin enthaltene Salz von o-Acetylsalicylsäure mit einer basischen Aminosäure bei einer mit einem Malvern 2600D-Gerät unter Standardbedingungen gemessenen Korngrößenverteilung ein eine mittlere Korngröße oberhalb einer Korngröße von 170 µm und einen Anteil von mehr als 70 % der Teilchen mit einer Korngröße in einem Bereich von 100 bis 200 µm aufweist. Das Salz weist üblicherweise einen Restfeuchtegehalt von weniger als 0,4 %, vorzugsweise von weniger als 0,3 % und insbesondere von weniger als 0,15 % Wasser auf. Der geringe Restfeuchtegehalt führt zu einer verbesserten Stabilität der erfindungsgemäßen Zusammensetzungen und Arzneimittel. Dem o-Acetylsalicylat kann ein gewisser Anteil Glycin zugesetzt werden, wie nachfolgend zum Herstellungsverfahren noch detailliert ausgeführt wird.

Die erfindungsgemäß als Komponente des Salzes von o-Acetylsalicylsäure geeigneten basischen Aminosäuren können in der L- oder in der D-Konfiguration auftreten oder auch als Gemisch von D- und L-Form. Der Begriff "Aminosäuren" bezeichnet erfindungsgemäß insbesondere die in der Natur vorkommenden α-Aminosäuren, umfasst darüber hinaus aber auch deren Homologe, Isomere und Derivate. Als Beispiel für Isomere können Enantiomere genannt werden. Derivate können beispielsweise mit Schutzgruppen versehene Aminosäuren sein. Als typische Beispiele für basische Aminosäuren seien genannt: Lysin, Arginin, Ornithin, Diaminobuttersäure. Das Salz der Acetylsalicylsäure mit Lysin ist besonders gut geeignet.

Unter Fließmittel werden erfindungsgemäß die oft auch Rieselhilfen genannten Hilfsstoffe verstanden, die pulverförmigen oder granulierten, insbesondere hygroskopischen Substanzen beigemischt werden, um deren Verklumpen oder Zusammenbacken zu verhindern und so dauernd freies Fließen (Fluidifikation) zu gewährleisten.

Bevorzugte Fließmittel sind hochdisperses Siliziumdioxid, und Saccharide sowie deren Mischungen. Besonders bevorzugte Fließmittel sind die Saccharide Mannitol, Sorbitol, Xylitol und Lactose sowie deren Mischungen. Die Fließmittel werden üblicherweise in einer Menge, bezogen auf die Menge an o-Acetylsalicylat, zwischen 1 und 70 Gew.-%, vorzugsweise zwischen 1 und 50 Gew.-%, eingesetzt. Die Menge an Fließmittel kann aber bei Bedarf auch größer sein. So kann in einer besonderen Ausführungsform der Erfindung bei einem aus einer erfindungsgemäßen Zusammensetzungen hergestellten Arzneimittel, beispielsweise in Form einer Kautablette, der Gehalt, bezogen auf das Arzneimittel, an Fließmittel, insbesondere an Sacchariden, bis zu 70 Gew.-% und/oder das 1- bis 2- oder sogar 2,5-fache der Gewichtsmenge an o-Acetylsalicylat betragen.

Zur Granulierung mittels Trockengranulation eignen sich erfindungsgemäß die bekannten, herkömmlichen Verfahren, insbesondere die Walzenkompaktierung. Die erfindungsgemäße Zusammensetzung ist demgemäß in einer bevorzugten Ausführungsform walzenkompaktiert. Bei der Granulierung können auch zur Verbesserung des Zwischenproduktes Hilfsstoffe eingesetzt werden (z.B. Bindemittel, Lösemittel, Saccharide, Polysaccharide).

Das erfindungsgemäße Arzneimittel ist im einfachsten Fall eine erfindungsgemäße Zusammensetzung, bestehend aus dem o-Acetylsalicylat und einem Fließmittel oder aus dem granulierten o-Acetylsalicylat.

Das erfindungsgemäße Arzneimittel wird vorzugsweise als einzeln dosierte feste oralen Darreichungsform ausgeführt, insbesondere als Tablette, Kautablette, Trinktablette, enteric-coated Tablette, Kapsel oder colon targeted Formulierung.

Zur Ausführung als Kapsel eignen sich sowohl die üblichen HartgelatineSteckkapseln als auch Steckkapseln aus HPMC (Methylhydroxypropylcellulose). Die HPMC-Steckkapseln lassen sich direkt oder nach Trocknung einsetzten. Überraschenderweise sind die o-Acetylsalicylate nicht nur in den HPMC-Steckkapseln sondern auch in den Hartgelatinesteckkapseln stabil. Dies demonstriert erneut die Stabilität der erfindungsgemäßen Zusammensetzungen und Arzneimittel. Weiterhin sind auch Steckkapseln die aus anderen Polymeren (z.B. Stärke, Cellulose, Hydroxypropylcellulose, Hydroxypropylcelluloselactat, Hydroxypropylcelluloseglycolid und Hydroxyethylhydroxypropylcellulose) geeignet.

Das erfindungsgemäße Arzneimittel kann neben dem o-Acetylsalicylat außer dem Fließmittel und Glycin weitere Hilfs- und/oder Wirkstoffe enthalten. Die weiteren Hilfs- und/oder Wirkstoffe können bei der Herstellung des Arzneimittels aus einer erfindungsgemäßen Zusammensetzung zugesetzt werden; sie können aber auch ganz oder teilweise bereits bei der Herstellung der erfindungsgemäßen Zusammensetzung, wobei auch sie dann gegebenenfalls granuliert werden, zugesetzt werden, so dass sie dann bereits von der erfindungsgemäßen Zusammensetzung umfasst sind.

So kann beispielsweise zur Tablettierung als FST-Mittel Magnesiumstearat in einer Menge von üblicherweise bis zu 2 Gew.-% zugesetzt werden. Überraschenderweise wird die Stabilität der Formulierung dadurch nicht herabgesetzt. Wird kein Magnesiumstearat hinzugefügt, so erfolgt die Formentrennung in der Tablettenpresse mittels der sogenannten externen Schmierung. Dabei wird Magnesiumstearat in kleinsten Mengen auf die Stempel und die Matrizenwand aufgesprüht. Der Kontakt zwischen dem Wirkstoff o-Acetylsalicylat und dem Magnesiumstearat wird dadurch stark herabgesetzt. Es können auch weitere FST-Mittel eingesetzt werden, z.B. Fumarsäure und/oder Adipinsäure.

In einer besonderen Ausführungsform enthält das erfindungsgemäße Arzneimittel als Hilfsstoffe ausschließlich wasserlösliche Hilfsstoffe, vorzugsweise die vorstehend als Fließmittel beschriebenen Saccharide, insbesondere ausgewählt aus der Gruppe, bestehend aus Mannitol, Sorbitol, Xylitol und Lactose sowie deren Mischungen. Durch die Wahl ausschließlich wasserlöslicher Hilfsstoffe lassen sich neben Tabletten und Kautabletten auch Trinktabletten herstellen. Diese lösen sich schnell und rückstandsfrei in Wasser auf und ergeben eine klare Lösung. Dies ist auch die ideale Basis für FDT (Fast Dissolve Tablets)-Zubereitungen. In einer weiteren Ausführungsform der Erfindung ist das Arzneimittel dem gemäß vollständig in Wasser löslich.

In noch einer besonderen Ausführungsform ist das erfindungsgemäße Arzneimittel frei von Brausemischungen.

Die Arzneimittel können weiterhin Farbstoffe (z.B. anorganische Pigmente wie Eisenoxide) sowie Geschmacks- und/oder Geruchskorngentien, insbesondere Süßungsmittel (z.B. Aspartam, Saccharin und/oder Acesulfam), enthalten.

Die Arzneimittel können in einer besonderen Ausführungsform der Erfindung außer Salzen von o-Acetylsalicyclsäure mit basischen Aminosäuren auch einen oder mehrere weitere pharmazeutische Wirkstoffe in wirksamen Mengen enthalten, insbesondere einen oder mehrere ADP-Rezeptorantagonisten (z.B. Ticlopidin und Clopidogrel), GPIIb/IIIa-Rezeptorantagonisten (z.B. Abciximab, Eptifabitide, Tirofiban, Orofiban, Xemilofiban und Sibrafiban), Phosphodiesterasehemmer (z.B. Dipyridamol), Thrombin-Rezeptorantagonisten (z.B. Hirudin, Hirulog und Argatroban), Faktor Xa-Inhibitoren (z.B. Antistatin, DX-9065 und Pentasaccharide), HMG-CoA-Rezeptorantagonisten (z.B. Cerivastatin, Simvastatin) und/oder Calciumantagonisten (z.B. Nifedipin).

Die erfindungsgemäßen Arzneimittel können als Analgetikum, Antipyretikum, Antirheumatikum, sowie als nichtsteroidale entzündungshemmende Arzneimittel beispielsweise zur Behandlung von Erkrankungen des rheumatischen Formenkreises, Arthritiden, Neuralgien, Myalgien und/oder Migräne eingesetzt werden. Insbesondere können sie aber auch als Thrombozytenaggregationshemmer in der Prävention und Therapie kardio- und cerebrovaskulärer Erkrankungen eingesetzt werden, z.B. bei ischämischen Herzkrankheiten, Schlaganfall (Stroke), stabiler und instabiler Angina pectoris, myocardialem Infarkt (z.B. akutem Myokardinfarkt), Bypass-Operationen, PTCA (Perkutane Transluminale Coronar-Angioplastie) und/oder Stent-Implantation. Weitere Anwendungsgebiete sind die Stimulierung des Immunsystems bei HIV-Patienten und die Tumorprophylaxe (z.B. Kolon-, Oesophagus- oder Lungenkarzinom), Verlangsamung des kognitiven Verfalls bei dementiellem Syndrom (z.B. Alzheimer-Erkrankung), Hemmung der Gallensteinbildung sowie die Behandlung diabetischer Erkrankungen.

Im allgemeinen hat es sich sowohl in der Human- als auch in der Veterinärmedizin als vorteilhaft erwiesen, den oder die oben genannten Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe eines erfindungsgemäßen oralen Arzneimittels enthält den oder die oben genannten Wirkstoffe vorzugsweise in Mengen von etwa 1 bis etwa 80, insbesondere 2 bis 30 mg/kg Körpergewicht.

### Herstellung des o-Acetylsalicylats

Die erfindungsgemäßen o-Acetylsalicylate können nach folgendem Verfahren hergestellt werden. Alle Ausgangsverbindungen sind gewerblich erhältlich.

Lösungen der Reaktionspartner, d. h. von o-Acetylsalicylsäure und der entsprechenden Aminosäure, werden bei einer Temperatur unterhalb von 30°C, vorzugsweise von 20 bis 25°C, unter Normaldruck möglichst rasch zusammengegeben und zu einer homogenen Phase vermischt. Als Lösungsmittel für die Reaktionspartner kommen Wasser beziehungsweise mit Wasser mischbare organische Lösungsmittel wie beispielsweise Alkohole wie Methanol, Ethanol oder iso-Propanol, insbesondere Ethanol, Ether wie Tetrahydrofuran (THF) oder Ketone wie Aceton in Frage.

Die Reaktionspartner werden in solchen Mengen eingesetzt, dass die basische Aminosäure in leichtem Überschuss vorliegt. Bevorzugt ist ein Verhältnis von o-Acetylsalicylsäure zu Aminosäure von 1:1,05 bis 1:1,5, wobei ein Verhältnis von o-Acetylsalicylsäure zu Aminosäure von 1:1,05 bis 1:1,2 besonders bevorzugt ist.

Die o-Acetylsalicylsäurelösung sollte einen Gehalt von 1 bis 10 Gew.-%, vorzugsweise 5 bis 10 Gew.-% und insbesondere bevorzugt von 6 bis 8 Gew.-% o-Acetylsalicylsäure aufweisen. Die Lösung der basischen Aminosäure sollte einen Gehalt von 10 bis 40 Gew.-%, vorzugsweise 15 bis 35 Gew.-% und insbesondere bevorzugt von 20 bis 30 Gew.-% Aminosäure aufweisen.

Aus der so hergestellten homogenen Mischung erfolgt anschließend, gegebenenfalls unter Zusatz von Impfkristallen, unter Zugabe eines deutlichen Überschusses an Aceton im Vergleich zu den Reaktionspartnern, beispielsweise eines 20 bis 50 %-igen, vorzugsweise eines 30 bis 40 %-igen Überschusses, die Kristallisation des erfindungsgemäßen o-Acetylsalicylats. Es ist sehr wichtig, dass die Temperatur der Kristallisationsphase in möglichst engen Grenzen gehalten wird. Die Temperatur darf 40°C nicht übersteigen und sollte vorzugsweise unterhalb von 35°C gehalten werden. Erfindungsgemäß bevorzugt ist eine Temperatur unterhalb von 25°C, insbesondere von 0°C. Als Impfkristalle können Kristalle des gewünschten Produkts, beispielsweise Aspisol®-Kristalle, verwendet werden. Die Kristallisation erfolgt unter Normaldruck.

Ebenso wichtig ist bei dem Verfahren das Einhalten einer bestimmten Rührenergie während der Kristallisation. Die homogene Mischung der Ausgangsprodukte darf nur sanft gerührt werden. Die anzulegende Rührenergie sollte nicht größer als 0,1 W pro Liter Reaktionsmedium sein. Erfindungsgemäß bevorzugt ist eine angelegte Rührenergie von 0,04 bis 0,06 W pro Liter Reaktionsmedium. Als Rührer kommen alle herkömmlichen entsprechend regulierbaren Rührgeräte wie beispielsweise ein Rührwerkbehälter mit Stromstörer in Betracht.

Die Lösung sollte zur Kristallisation nicht länger als 20 Stunden unter den vorstehend angegebenen Bedingungen gehalten werden. Erfindungsgemäß bevorzugt ist eine Kristallisationsdauer von weniger als 10 Stunden unter den vorstehend angegebenen Bedingungen, wobei ein Zeitraum von 1 bis 8 Stunden besonders bevorzugt ist.

Auf Wunsch kann das erfindungsgemäße o-Acetylsalicylat auch Glycin enthalten. Die Menge an Glycin ist frei wählbar. Erfindungsgemäß bevorzugt ist ein Anteil, bezogen auf die Gesamtmenge von o-Acetylsalicylat und Glycin, von 5 bis 30 Gew.-%, besonders bevorzugt von 5 bis 15 Gew.-% und insbesondere bevorzugt von 10 Gew.-% Glycin, in der Reaktionslösung.

Das Glycin kann gemäß der vorliegenden Erfindung als Lösung in Wasser oder einem mit Wasser mischbaren organischen Lösungsmittel, wobei als organische Lösungsmittel die vorstehend beschriebenen Lösungsmittel verwendet werden können, der Reaktionsmischung aus den Reaktionspartnern zugegeben werden. Glycin verhält sich gegenüber diesen Reaktionspartnern inert. Unter den vorstehend genannten Bedingungen lassen sich dadurch Kristallisationsvorgänge der beiden Feststoffe (o-Acetylsalicylat und Glycin) aus der homogenen Phase führen (Cokristallisation).

Das Glycin kann aber auch in Form einer Suspension zu einer bereits kristallisierten Suspension des o-Acetylsalicylats gegeben werden. Die Glycin-Suspension kann auf herkömmliche Weise hergestellt werden. Erfindungsgemäß bevorzugt ist die Herstellung einer Glycin-Suspension aus einem Lösungsmittelgemisch aus Wasser und einem Alkohol wie beispielsweise Ethanol.

Die Art der Zugabe des Glycins hat keinen Einfluss auf die Eigenschaften des erfindungsgemäßen o-Acetylsalicylats. Es ist zu bemerken, dass der Zusatz von Glycin zu den erfindungsgemäßen o-Acetylsalicylaten nicht erforderlich ist. Insbesondere hat die Anwesenheit von Glycin keinen Einfluss auf die Stabilität der erfindungsgemäßen o-Acetylsalicylate.

Das Kristallisat wird anschließend auf herkömmliche Weise isoliert, beispielsweise durch Filtrieren oder Zentrifugieren. Der Feststoff wird mehrfach mit organischen Lösungsmitteln gewaschen, wobei erfindungsgemäß Alkohole wie beispielsweise Ethanol und/oder Ketone wie Aceton oder Mischungen von Alkoholen oder Ketonen, beispielsweise Mischungen von Ethanol und Aceton, oder die Verwendung verschiedener derartiger Lösungsmittel bevorzugt ist.

Der Feststoff wird anschließend unter vermindertem Druck getrocknet. Hierbei sollte die Temperatur unter 50°C, vorzugsweise unter 40°C und besonders bevorzugt unter 35°C gehalten werden. Es sollte ein Druck von weniger als 50 mbar, vorzugsweise von weniger als 30 mbar an den Feststoff angelegt werden. Die Trocknung kann unter herkömmlichen Bedingungen erfolgen, beispielsweise in einem Trockengerät.

Das Verfahren kann auch vollständig unter sterilen Bedingungen durchgeführt werden. Die hierfür erforderlichen Abweichungen von der obigen Vorgehensweise beispielsweise hinsichtlich Sterilisierung der Ausgangsverbindungen sowie der eingesetzten Apparaturen sind dem Fachmann bekannt.

### Beispiele

Die vorliegende Erfindung wird nachstehend anhand von bevorzugten Beispielen näher dargestellt. Soweit nicht anderweitig angegeben, beziehen sich alle Mengenangaben auf Gewichtsprozent.

### Beispiel 1: Lysinacetylsalicylat

In einem Rührwerkbehälter mit Stromstörer gibt man eine Lösung von 9,9 kg o-Acetylsalicylsäure in 120 kg Ethanol. Bei 20 bis 25°C fügt man innerhalb kurzer Zeit eine Lösung von 9,0 kg Lysinhydrat in 26,5 kg Wasser hinzu und mischt die Lösungen, so dass eine Temperatur von 30°C nicht überschritten wird. Es werden 50 g an Impfkristallen hinzugefügt und die bereits kristallisierende Mischung unter Kühlen auf 0°C mit 120 kg Aceton versetzt. Man lässt unter sanftem Rühren bei 0°C eine bis acht Stunden kristallisieren. Das Kristallisat wird auf einem Filter oder einer Zentrifuge isoliert. Das feuchte Produkt wird auf einem Trennapparat mehrfach mit Ethanol gewaschen. Das feuchte Produkt wird in einen Trockner überführt und darin bei einem Druck von weniger als 30 mbar bei einer Temperatur von nicht mehr als 40°C getrocknet.

Es wurden 89 bis 94 % des gewünschten Produkts erhalten, das einen Restfeuchtegehalt von 0,10 bis 0,15 % aufwies.

### Beispiel 2: D,L-Lysinacetylsalicylat mit 10 % Glycin

In einem Rührwerkbehälter mit Stromstörer gibt man eine Lösung von 9,9 kg o-Acetylsalicylsäure in 145 kg Ethanol. Bei 20 bis 25°C fügt man innerhalb kurzer Zeit eine Lösung von 9,0 kg D,L-Lysinhydrat und 2,4 kg Glycin in 35 kg Wasser hinzu und mischt die Lösungen, so dass eine Temperatur von 30°C nicht überschritten wird. Es werden 50 g an Impfkristallen hinzugefügt und die bereits kristallisierende Mischung unter Kühlen auf 0°C mit 120 kg Aceton versetzt. Man lässt unter sanftem Rühren bei 0°C eine bis acht Stunden kristallisieren. Das Kristallisat wird auf einem Filter oder einer Zentrifuge isoliert. Das feuchte Produkt wird auf einem Trennapparat mehrfach nacheinander mit Ethanol und Aceton gewaschen. Das feuchte Produkt wird in einen Trockner überführt und darin bei einem Druck von weniger als 30 mbar bei einer Temperatur von nicht mehr als 40°C getrocknet.

Es wurden 90 bis 95 % des gewünschten Produkts erhalten, das einen Restfeuchtegehalt von 0,10 bis 0,15 % aufwies.

### Beispiel 3: D,L-Lysinacetylsalicylat mit 10 % Glycin

In einem Rührwerkbehälter mit Stromstörer gibt man eine Lösung von 9,9 kg o-Acetylsalicylsäure in 120 kg Ethanol. Bei 20 bis 25°C fügt man innerhalb kurzer Zeit eine Lösung von 9,0 kg Lysinhydrat in 26,5 kg Wasser hinzu und mischt die Lösungen, so dass eine Temperatur von 30°C nicht überschritten wird. Es werden 50 g an Impfkristallen hinzugefügt und die bereits kristallisierende Mischung unter Kühlen auf 0°C mit 120 kg Aceton versetzt. Man lässt unter sanftem Rühren bei 0°C eine bis acht Stunden kristallisieren. In einem separaten Rührwerkbehälter wird eine Suspension von 2,1 kg Glycin in 8 kg Wasser und 25 kg Ethanol hergestellt. Diese wird in die Salicylatsuspension gegeben. Das Kristallgemisch wird auf einem Filter oder einer Zentrifuge isoliert. Das feuchte Produkt wird auf einem Trennapparat mehrfach mit Ethanol gewaschen. Das feuchte Produkt wird in einen Trockner überführt und darin bei einem Druck von weniger als 30 mbar bei einer Temperatur von nicht mehr als 40°C getrocknet.

Es wurden 89 bis 94 % des gewünschten Produkts erhalten, das einen Restfeuchtegehalt von 0,10 bis 0,15 % aufwies.

Das Produkt wurde gemäß den Beispielen 4 bis 6 zur Herstellung von Kautabletten, Tabletten und Kapseln verwendet.

### Bestimmung der Korngrößenverteilung

Das Lysinacetylsalicylat aus Beispiel 1 sowie handelsübliches Aspisol® (von der Firma Bayer AG vertrieben) wurden in einem Malvern 2600 D-Messgerät der Firma Malvern unter folgenden Standardbedingungen untersucht.

Das Messgerät Malvern 2600 besteht aus einem He/Ne-Laser, einer Messküvette mit thermostatisierbarem Vorratsbehälter-System, Fourier-Linsen und MultielementDetektor. Die gemessenen Lichtintensitäten werden in eine Korngrößenverteilung umgesetzt. Die Ausrichtung von Laser und Linse wird vor jeder Messung manuell eingestellt und das Meßgerät durch eine Leerwertmessung kontrolliert. Die Leerwertimpulse dürfen einen Höchstwert von 20 pro Detektorelement nicht überschreiten.

Die zu untersuchende Probe wird von Hand ca. 15 s geschüttelt; anschließend wird mit einem Spatel eine Probe entnommen. Die Probenmenge richtet sich nach dem zulässigen Obscuration-Bereich (0,1-0,3) des Messgerätes. Die entnommene Probe wird mit einem üblichen Dispergiermittel wie Baysilon M10® (Firma Bayer AG) schonend (durch Einrühren mit einem Glasstab) in einem Becherglas vordispergiert und anschließend in den Vorratsbehälter des Messgerätes, welcher ebenfalls mit dem Dispergiermittel gefüllt ist, eingefüllt. Das Becherglas wird mit dem Dispergiermittel vollkommen ausgespült, um die repräsentative Probenahme zu gewährleisten.

Die Messung erfolgt mit einer eingestellten Brennweite von 300 mm, einer Thermostatisierung von 20°C und einem zulässigen Obscuration-Bereich von 0,1-0,3.

Das Produkt wird nach Ultraschallzeiten von 0, 15 und 60 Sekunden vermessen. Der Ultraschallfinger befindet sich dazu im Vorratsgefäß des Produktumlaufs. Die Suspension wird in einem geschlossenen Kreislauf durch die Messküvette gepumpt. Die vom Detektor registrierten Signale werden ausgewertet und in die Korngrößenverteilung umgerechnet.

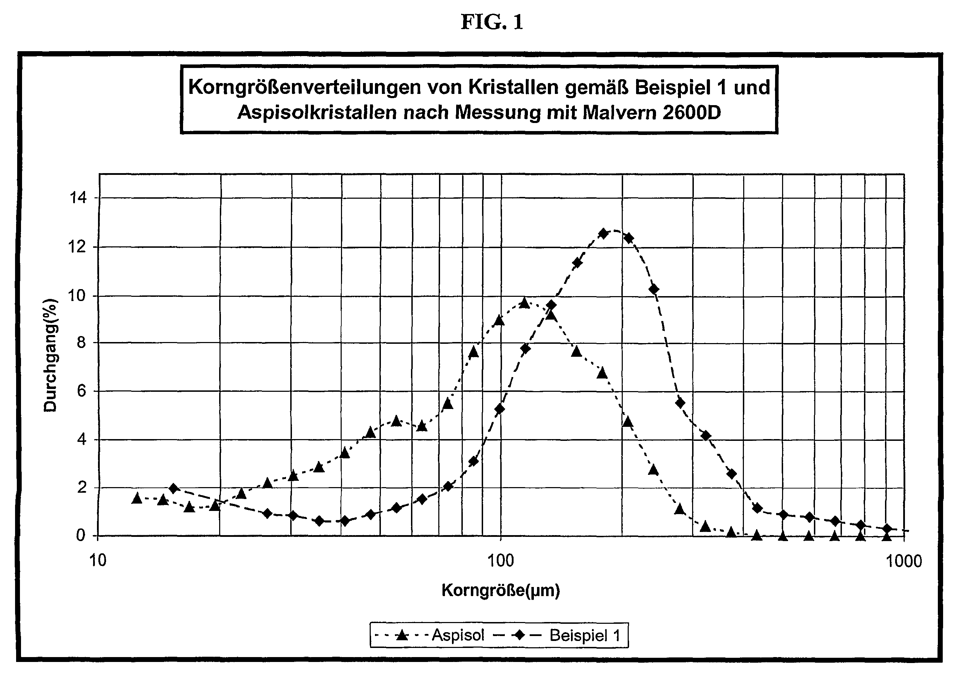

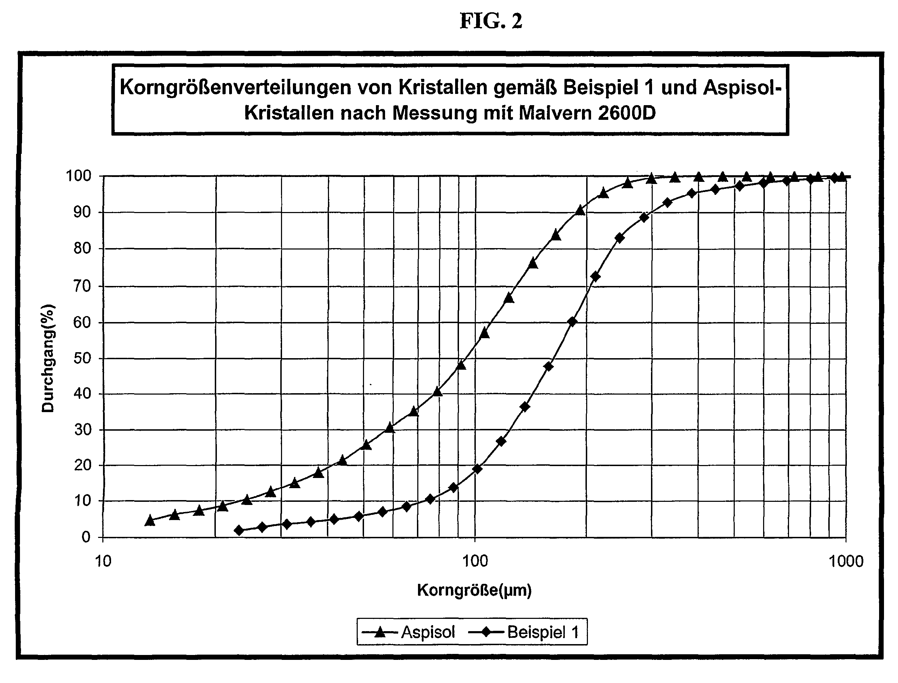

Die so erhaltenen Ergebnisse sind in Fig. 1 und 2 dargestellt.

### Beispiel 4: Kautabletten

In diesem Beispiel wird die Herstellung von Kautabletten gemäß der vorliegenden Erfindung beschrieben. Neben der Verbesserung der Fließeigenschaften verbessern die eingesetzten Saccharide den Geschmackseindruck. Die Kombination zweier Süßungsmittel bewirkt eine Geschmacksmaskierung.

Die Wirk- und Hilfsstoffe wurden eingewogen, 10 Minuten in einem Turbulamischer gemischt und für 12 - 24 Stunden mit trockener Luft zur Trocknung durchströmt. Die Kompaktierung erfolgte auf einer handelsüblichen Tablettenpresse. Sie kann auf allen handelsüblichen Tablettenpressen (z.B. Exzenter- und Rundläufertablettenpressen) erfolgen.

| Einsatzstoff | Menge pro Tablette [mg] | Menge pro Tablette [mg] |
|---|---|---|
| Zusammensetzung aus Bsp. 3 | 1000,00 | 1000,00 |
| Xylitol | 460,00 | - |
| Sorbitol | - | 460,00 |
| Magesiumstearat | 32,00 | 32,00 |
| Aspartam | 4,00 | 4,00 |
| Saccharin | 4,00 | 4,00 |
| Tablettenmasse [mg] | 1500,00 | 1500,00 |

Die Tabletten können in wasserdampfdichte Verbundfolien (z.B. PAP-Surlyn-Verbundfolien) oder in Alu-Alu-Blister eingeschweißt werden.

### Beispiel 5: Tabletten

In diesem Beispiel wird die Herstellung von Tabletten gemäß der vorliegenden Erfindung beschrieben. Neben der Verbesserung der Fließeigenschaften verbessern die eingesetzten Saccharide den Geschmackseindruck.

Die Wirk- und Hilfsstoffe wurden eingewogen, 10 Minuten in einem Turbulamischer gemischt und für 12 - 24 Stunden mit trockener Luft zur Trocknung durchströmt. Die Kompaktierung erfolgte auf einer handelsüblichen Tablettenpresse mit externer Schmierung. Sie kann auf allen handelsüblichen Tablettenpressen (z.B. Exzenter- und Rundläufertablettenpressen) mit externer Schmierung erfolgen.

| Einsatzstoff | Menge pro Tabl. | Menge pro Tabl. | Menge pro Tabl. |
|---|---|---|---|
| | [mg] | [mg] | [mg] |
| Zusammensetzung aus | 200,00 | 200,00 | 200,00 |
| Bsp. 3 | | | |
| Sorbitol | 40,00 | - | - |
| Xylitol | - | 40,00 | - |
| Mannitol | - | - | 40,00 |
| Tablettenmasse [mg] | 240,00 | 240,00 | 240,00 |

Die Tabletten können in wasserdampfdichte Verbundfolien (z.B. PAP-Surlyn-Verbundfolien) oder in Alu-Alu-Blister eingeschweißt werden.

Da die Tabletten nur wasserlösliche Wirk- und Hilfsstoffe enthalten, können die erfindungsgemäß hergestellten Tabletten auch als Trinktabletten eingesetzt und appliziert werden.

### Beispiel 6: Kapseln

In diesem Beispiel wird die Herstellung von Kapseln gemäß der vorliegenden Erfindung beschrieben. Die eingesetzten Hilfsstoffe dienen der Verbesserung der Fließeigenschaften. Die Menge der eingesetzten Hilfsstoffe richtet sich nach der Kapselgröße und ist jeweils individuell anzupassen. Eingesetzt wurden Steckkapselhüllen aus Hartgelatine und aus Methylhydroxypropylcellulose unterschiedlicher Größen. Die eingesetzten Kapselhüllen können auch vor der Befüllung getrocknet und so deren Gehalt an freiem Wasser reduziert werden.

Die Wirk- und Hilfsstoffe wurden unter Berücksichtigung der Kapselgröße eingewogen, 10 Minuten in einem Turbulamischer gemischt und für 12 - 24 Stunden mit trockener Luft zur Trocknung durchströmt. Die Verkapselung erfolgte auf einer handelsüblichen Kapselfüllmaschine. Sie kann auf allen handelsüblichen Kapselfüllmaschinen (sowohl manuellen als auch automatischen Kapselfüllmaschinen) erfolgen.

| Einsatzstoff | Menge pro Kapsel | Menge pro Kapsel |
|---|---|---|
| | [mg] | [mg] |
| Zusammensetzung aus Bsp. 3 | 200 mg | 200 mg |
| Mannitol | q. s. | q. s. |
| Kapselmaterial | HPMC-Kapseln | Hartgelatinekapseln |

Die Kapseln können in wasserdampfdichte Verbundfolien (z.B. PAP-Surlyn-Verbundfolien) oder in Alu-Alu-Blister eingeschweißt werden.

### Bestimmung der Stabilität

Die Stabilität der hergestellten erfindungsgemäßen einzeln dosierten festen oralen Arzneimittel der Beispiele 4 bis 6 wurde nach Einlagerung dieser bei einer Temperatur von 25°C und einer relativen Luftfeuchte von 60 % bestimmt. Als Indikator für die Stabilität der Zubereitungen wurde der Gehalt an Salicylsäure als Abbauprodukt des o-Acetylsalicylates bestimmt.

### Stabilität der Formulierungen

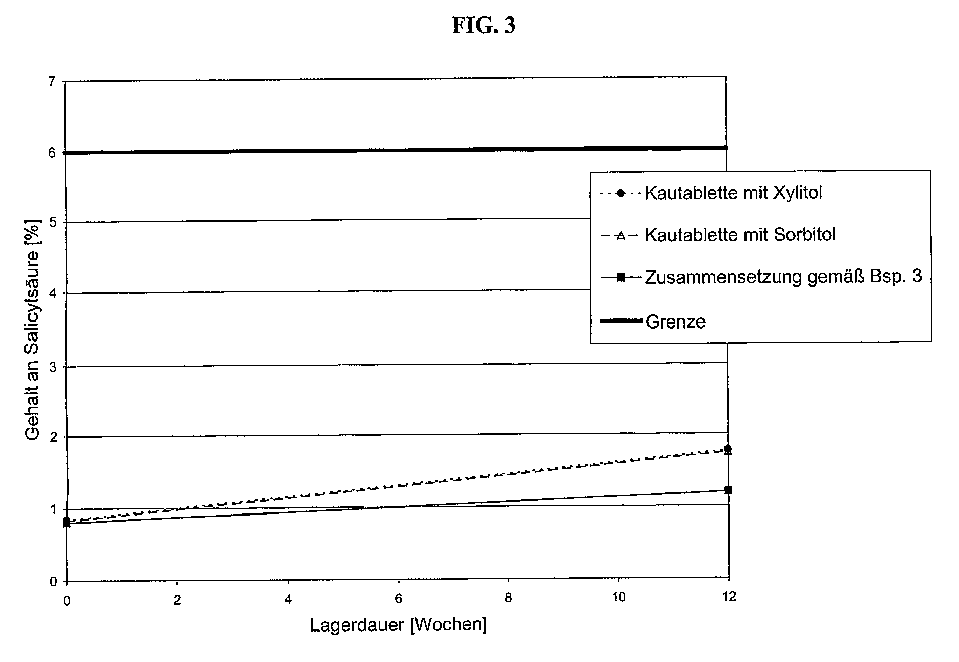

Die Stabilität der Formulierungen ist in den Diagrammen der Fig. 3 bis 5 dargestellt. Es ist daraus ersichtlich, dass die erfindungsgemäßen Zubereitungen eine ausreichende Haltbarkeit besitzen und bezüglich der Abbaugeschwindigkeit mit dem o-Acetylsalicylsäuresalz mit Lysin vergleichbar sind.

In Fig. 3 ist der Abbau von o-Acetylsalicylat zu Salicylsäure in Kautabletten gemäß Beispiel 4 bei Lagerung bei 25°C und 60 % relativer Feuchte im Vergleich zur Zusammensetzung gemäß Beispiel 3 dargestellt.

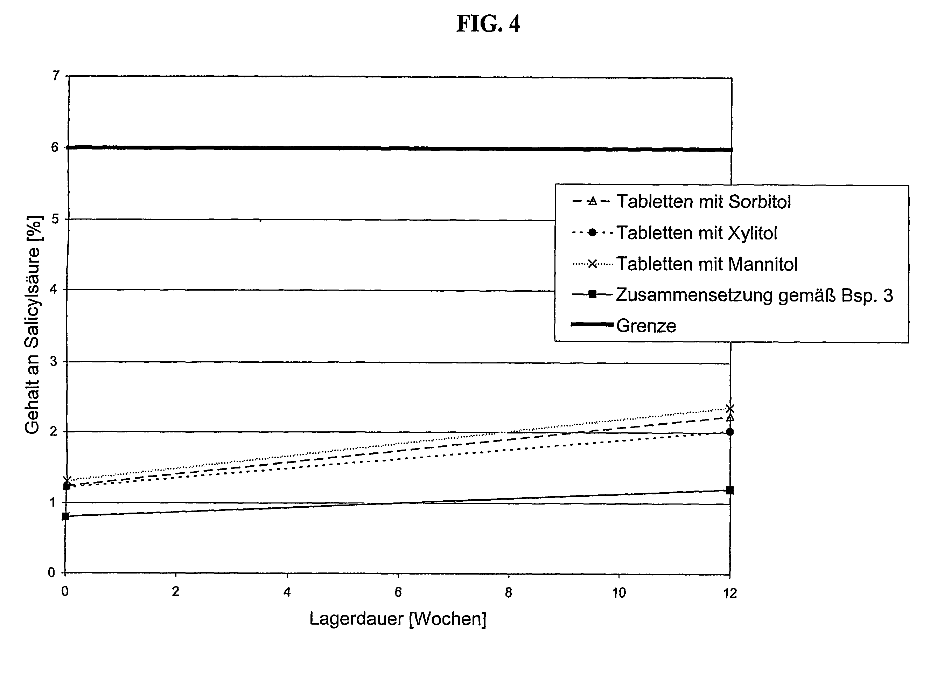

In Fig. 4 ist der Abbau von o-Acetylsalicylat zu Salicylsäure in Tabletten gemäß Beispiel 5 bei Lagerung bei 25°C und 60 % relativer Feuchte im Vergleich zur Zusammensetzung gemäß Beispiel 3 dargestellt.

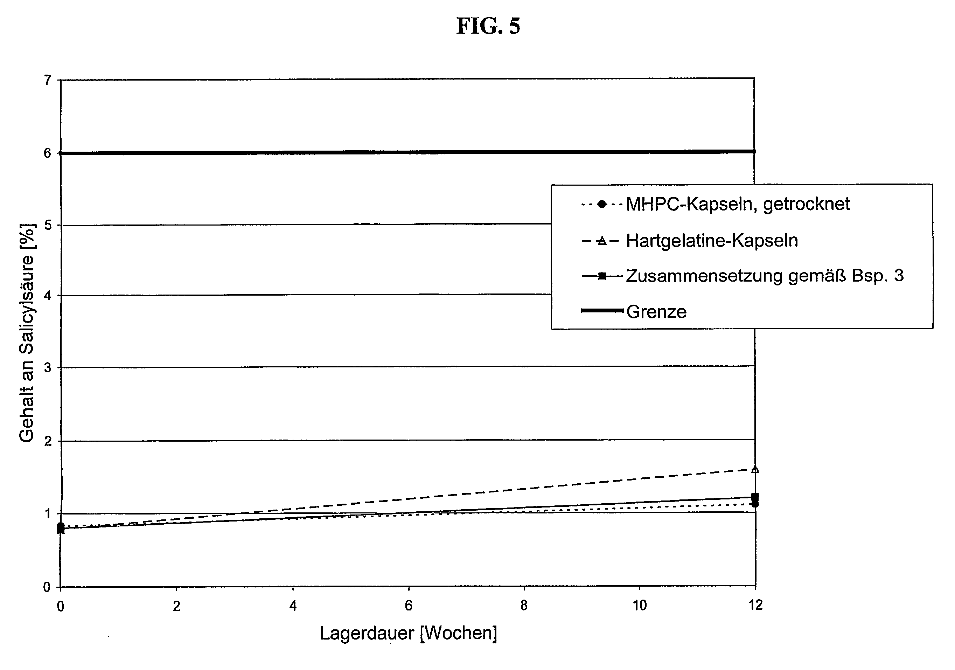

In Fig. 5 ist der Abbau von o-Acetylsalicylat zu Salicylsäure in Kapseln gemäß Beispiel 6 bei Lagerung bei 25°C und 60 % relativer Feuchte im Vergleich zur Zusammensetzung gemäß Beispiel 3 dargestellt.

## Patentansprüche

1. Zusammensetzung, umfassend ein Salz von o-Acetylsalicylsäure mit einer basischen Aminosäure, das bei einer mit einem Malvern 2600D-Gerät unter Standardbedingungen gemessenen Korngrößenverteilung eine mittlere Korngröße oberhalb einer Korngröße von 160 µm und einen Anteil von mehr als 60 % der Teilchen mit einer Korngröße in einem Bereich von 100 bis 200 µm aufweist, **dadurch gekennzeichnet, dass** die Zusammensetzung zusätzlich ein Fließmittel umfasst und/oder mittels Trockengranulation granuliert ist, wobei mikrokristalline Cellulose als Fließmittel ausgenommen ist.

2. Zusammensetzung nach Anspruch 1, **dadurch gekennzeichnet, dass** sie als Fließmittel ein oder mehrere Saccharide, vorzugsweise ausgewählt aus der Gruppe, bestehend aus Mannitol, Sorbitol, Xylitol und Lactose sowie deren Mischungen, umfasst.

3. Zusammensetzung nach Anspruch 2, **dadurch gekennzeichnet, dass** sie walzenkompaktiert, ist.

4. Zusammensetzung nach einem der vorstehenden Ansprüche, **dadurch gekennzeichnet, dass** das Salz bei einer mit einem Malvern 2600D-Gerät unter Standardbedingungen gemessenen Korngrößenverteilung eine mittlere Korngröße oberhalb einer Korngröße von 170 µm und einen Anteil von mehr als 70 % der Teilchen mit einer Korngröße in einem Bereich von 100 bis 200 µm aufweist.

5. Zusammensetzung nach einem der vorstehenden Ansprüche, **dadurch gekennzeichnet, dass** die basische Aminosäure Lysin, Arginin, Histidin, Ornithin oder Diaminobuttersäure, vorzugsweise Lysin, ist.

6. Zusammensetzung nach einem der vorstehenden Ansprüche, **dadurch gekennzeichnet, dass** sie zusätzlich einen Anteil von 5 bis 15 Gew.-% Glycin, bezogen auf die Gesamtmenge von o-Acetylsalicylat und Glycin, umfasst.

7. Arzneimittel, enthaltend mindestens eine Zusammensetzung nach einem der vorstehenden Ansprüche.

8. Arzneimittel nach dem vorstehenden Anspruch, **dadurch gekennzeichnet, dass** es als einzeln dosierte feste oralen Darreichungsform, insbesondere als Tablette, Kautablette, Trinktablette, enteric-coated Tablette, Kapsel oder colon targeted Formulierung, ausgeführt ist.

9. Arzneimittel nach Anspruch 7 oder 8, **dadurch gekennzeichnet, dass** es nur wasserlösliche Hilfsstoffe, vorzugsweise Fließmittel gemäß Anspruch 2, enthält.

10. Arzneimittel nach einem der Ansprüche 7 bis 9, **dadurch gekennzeichnet, dass** es vollständig in Wasser löslich ist.

11. Arzneimittel nach einem der Ansprüche 7 bis 10, **dadurch gekennzeichnet, dass** es einen oder mehrere weitere pharmazeutische Wirkstoffe, insbesondere einen oder mehrere ADP-Rezeptorantagonisten, GPIIb/IIIa-Rezeptorantagonisten, Phosphodiesterasehemmer, Thrombin-Rezeptorantagonisten, Faktor Xa-Inhibitoren, HMG-CoA-Rezeptorantagonisten und/oder Calciumantagonisten enthält.

12. Verwendung von Zusammensetzungen gemäß einem der Ansprüche 1 bis 6 zur Herstellung eines Arzneimittels zur Behandlung von Erkrankungen des rheumatischen Formenkreises, Arthritiden, Neuralgien, Myalgien und/oder Migräne.

13. Verwendung von Zusammensetzungen gemäß einem der Ansprüche 1 bis 6 zur Herstellung eines Arzneimittels zur Behandlung von ischämischen Herzkrankheiten, Schlaganfall, Angina pectoris, myocardialem Infarkt, Bypass-Operationen, PTCA und/oder Stent-Implantation.

14. Verwendung von Zusammensetzungen gemäß einem der Ansprüche 1 bis 6 zur Herstellung eines Arzneimittels zur Stimulierung des Immunsystems bei HIV-Patienten, Tumorprophylaxe, Verlangsamung des kognitiven Verfalls bei dementiellem Syndrom, Hemmung der Gallensteinbildung und/oder Behandlung diabetischer Erkrankungen.

## Claims

1. Composition, comprising a salt of O-acetylsalicylic acid with a basic amino acid, which salt has an average particle size above a particle size of 160 µm and a proportion of more than 60% of the particles having a particle size in a range from 100 to 200 µm in a particle size distribution measured using a Malvern 2600D apparatus under standard conditions, **characterized in that** the composition additionally comprises a flow improver and/or is granulated by dry granulation, excluding microcrystalline cellulose as flow improver.

2. Composition according to Claim 1, **characterized in that** it comprises, as flow improver, one or more saccharides, preferably selected from the group consisting of mannitol, sorbitol, xylitol and lactose and their mixtures.

3. Composition according to Claim 2, **characterized in that** it is roller-compacted.

4. Composition according to any of the preceding claims, **characterized in that** the salt has an average particle size above a particle size of 170 µm and a proportion of more than 70% of the particles having a particle size in a range from 100 to 200 µm in a particle size distribution measured using a Malvern 2600D apparatus under standard conditions.

5. Composition according to any of the preceding claims, **characterized in that** the basic amino acid is lysine, arginine, histidine, ornithine or diaminobutyric acid, preferably lysine.

6. Composition according to any of the preceding claims, **characterized in that** it additionally comprises a proportion of from 5 to 15% by weight of glycine, based on the total amount of O-acetylsalicylate and glycine.

7. Pharmaceutical, comprising at least one composition according to any of the preceding claims.

8. Pharmaceutical according to the preceding claim, **characterized in that** it is provided as a single-dose solid oral administration form, in particular as a tablet, a chewable tablet, a soluble tablet, an enteric-coated tablet, a capsule or a colon-targeted formulation.

9. A pharmaceutical as claimed in Claim 7 or 8, **characterized in that** it only comprises water-soluble auxiliaries, preferably flow improvers as set forth in Claim 2.

10. Pharmaceutical according to any of Claims 7 to 9, **characterized in that** it is completely soluble in water.

11. Pharmaceutical according to any of Claims 7 to 10, **characterized in that** it comprises one or more further pharmaceutically active compounds, in particular one or more ADP receptor antagonists, GPIIb/IIIa receptor antagonists, phosphodiesterase inhibitors, thrombin receptor antagonists, factor Xa inhibitors, HMG-CoA receptor antagonists and/or calcium antagonists.

12. Use of compositions according to any of Claims 1 to 6 for preparing a pharmaceutical for treating disorders of a rheumatic type, arthritis, neuralgia, myalgia and/or migraine.

13. Use of compositions according to any of Claims 1 to 6 for preparing a pharmaceutical for treating ischaemic heart diseases, stroke, angina pectoris, myocardial infarction, bypass operations, PTCA and/or stent implants.

14. Use of compositions according to any of Claims 1 to 6 for preparing a pharmaceutical for stimulating the immune system of HIV patients, for tumour prophylaxis, for slowing down the cognitive deterioration associated with dementia, for inhibiting the formation of gallstones and/or for treating diabetic disorders.

## Revendications

1. Composition comprenant un sel d'acide o-acétylsalicylique d'un acide aminé basique, qui présente, pour une répartition granulométrique mesurée dans des conditions normalisées avec un appareil Malvern 2600D, une granulométrie moyenne au-dessus d'une valeur de 160 µm et une proportion de plus de 60 % des particules ayant une granulométrie comprise dans une plage de 100 à 200 µm, composition **caractérisée en ce qu'**elle comprend en outre un agent d'écoulement et/ou est granulée par granulation à sec, la cellulose microcristalline en tant qu'agent d'écoulement étant exclue.

2. Composition suivant la revendication 1, **caractérisée en ce qu'**elle comprend comme agent d'écoulement un ou plusieurs saccharides, avantageusement choisis dans le groupe comprenant le mannitol, le sorbitol, le xylitol et le lactose ainsi que leurs mélanges.

3. Composition selon la revendication 2, **caractérisée en ce qu**'elle est compactée au rouleau.

4. Composition suivant l'une des revendications précédentes, **caractérisée en ce que** le sel présente, pour une répartition granulométrique, mesurée dans des conditions normalisées avec un appareil Malvern 2600D, une granulométrie moyenne au-dessus d'une valeur de 170 µm et une proportion de plus de 70 % des particules ayant une granulométrie comprise dans une plage de 100 à 200 µm.

5. Composition suivant l'une des revendications précédentes, **caractérisée en ce que** l'acide aminé basique est la lysine, l'arginine, l'histidine, l'ornithine ou l'acide diaminobutyrique, avantageusement la lysine.

6. Composition suivant l'une des revendications précédentes, **caractérisée en ce qu'**elle comprend en outre une proportion de 5 à 15 % en poids de glycine, par rapport à la quantité totale de o-acétylsalicylate et de glycine.

7. Médicament contenant au moins une composition suivant l'une des revendications précédentes.

8. Médicament suivant la revendication précédente, **caractérisé en ce qu'**il est réalisé comme forme d'administration orale solide dosée individuellement, en particulier comme comprimé, comprimé à mâcher, comprimé pour boisson, comprimé à délitement entérique, gélule ou formulation ciblée sur le côlon.

9. Médicament suivant la revendication 7 ou 8, **caractérisé en ce qu'**il ne contient que des substances auxiliaires solubles dans l'eau, avantageusement des agents d'écoulement selon la revendication 2.

10. Médicament suivant l'une des revendications 7 à 9, **caractérisé en ce qu'**il est entièrement soluble dans l'eau.

11. Médicament suivant l'une des revendications 7 à 10, **caractérisé en ce qu'**il contient une ou plusieurs autres substances pharmaceutiques actives, en particulier un ou plusieurs antagonistes des récepteurs de l'ADP, antagonistes des récepteurs GPIIb/IIIa, inhibiteurs de phosphodiestérase, antagonistes des récepteurs de thrombine, inhibiteurs du facteur Xa, antagonistes des récepteurs de HMG-CoA et/ou des antagonistes du calcium.

12. Utilisation de compositions suivant l'une des revendications 1 à 6, pour la préparation d'un médicament destiné au traitement de maladies de l'ensemble des formes rhumatismales, d'arthrites, de névralgies, de myalgies et/ou de la migraine.

13. Utilisation de compositions suivant l'une des revendications 1 à 6, pour la préparation d'un médicament destiné au traitement d'affections cardiaques ischémiques, de l'apoplexie cérébrale, de l'angine de poitrine, de l'infarctus du myocarde, d'opérations de pontage, de PTCA et/ou de l'implantation d'extenseurs.

14. Utilisation de compositions suivant l'une des revendications 1 à 6, pour la préparation d'un médicament destiné à la stimulation du système immunitaire chez des patients atteints du virus HIV, à la prophylaxie de tumeurs, au ralentissement de la dégradation cognitive en cas de syndrome de démence, à l'inhibition de la formation de calculs biliaires et/ou au traitement d'affections diabétiques.
